# EUROPEAN PATENT SPECIFICATION

(11) **EP 3 708 204 B1**
(45) Date of publication and mention of the grant of the patent: **04.12.2024**
(21) Application number: 19885559.5
(22) Date of filing: 18.10.2019
(51) Int. Cl.: A61M 5/28, A61J 1/06, A61M 5/32, A61M 5/315

(54) **SYRINGE SUITABLE FOR HYDROGEN PEROXIDE SOLUTION, AND KIT**
FÜR WASSERSTOFFPEROXIDLÖSUNG GEEIGNETE SPRITZE UND KIT
SERINGUE APPROPRIÉE POUR UNE SOLUTION DE PEROXYDE D'HYDROGÈNE ET NÉCESSAIRE

(30) Priority: 16.11.2018 JP 2018215913; 12.02.2019 JP 2019022388; 21.06.2019 JP 2019115440
(43) Date of publication of application: 16.09.2020
(60) Divisional application: 24203155.7
(73) Proprietor: KORTUC Inc., Tokyo 1056004 (JP)
(72) Inventor: YAMASHITA, Shogo, Tokyo 1056004 (JP)
(74) Representative: Gulde & Partner
(86) International application number: PCT/JP2019/041116
(87) International publication number: WO 2020/100525

(56) References cited:
- WO-A1-2008/041514
- WO-A1-2010/024209
- WO-A1-2014/187779
- WO-A1-2015/030063
- WO-A1-2018/070136
- JP-A- 2002 191 695
- JP-A- 2004 537 340
- JP-A- 2014 057 632
- JP-A- H07 250 898
- JP-A- H07 250 898
- JP-A- H08 206 118
- JP-A- H08 276 013
- RU-C1- 2 589 682
- US-A1- 2003 012 746
- US-A1- 2017 065 772

## Description

### TECHNICAL FIELD

The present invention relates to a pre-filled syringe, and particularly to a syringe made of a material having a lower rate of metal ion elution in the presence of a hydrogen peroxide solution compared with glass.

### BACKGROUND

A hydrogen peroxide solution is used industrially as a bleaching agent, and as a disinfectant in the food industry. A hydrogen peroxide solution containing 2.5 to 3.5% (w/v) hydrogen peroxide (known as "oxydol" in the Japanese Pharmacopoeia) is used for medical purposes as a disinfectant.

This hydrogen peroxide solution can be used as a radiosensitizer by mixing it with a solution of hyaluronic acid or a salt thereof such as sodium hyaluronate in a pre-determined ratio, and then injecting the mixture into a tumor just before the therapeutic radiation dose (WO 2008/041514 A).

JP H07-250898 A discloses a syringe comprising a needle assembly including a cap made of a thermoplastic. WO 2018/070136 A1 discloses a pre-filled syringe set comprising a dose adjustment tool.US 2017/0065772 A1 discloses a segmented safety cover for protecting a needle cannula of a syringe. The segmented safety cover comprises a needle hub, a proximal cover segment, a distal cover segment and a distal end cap. The various segments of the cover may comprise polymeric materials (thermoplastic or thermoset), metallic materials, ceramics, composites or the like.

WO 2014/187779 A1 discloses a primary container such as a syringe made of glass or a plastic (e.g. cycloolefin or polypropylene) pre-filled with an injectable pharmaceutical composition. The primary container is contained in a functional packaging material permeable for a sterilization gas, such as gaseous hydrogen peroxide or ethylene oxide. Sterilization of an outer surface of the primary container is achieved by subjecting the package to the sterilization gas.

WO 2010/0024209 A1 discloses a syringe sterilized with hydrogen peroxide gas.

US 2003/012746 A1 discloses a pre-filled syringe containing a hydrogen peroxide bleaching solution.

### SUMMARY OF THE INVENTION

### PROBLEMS TO BE SOLVED BY THE INVENTION

Because hydrogen peroxide decomposes rapidly when removed from a special storage container that shields it from light, it must be drawn out in the appropriate volume or weight and then mixed with the sodium hyaluronate solution just before injection, when used as a radiation sensitizer as in WO 2008/041514 A. This places an extra burden on the medical personnel treating the patient. Either the hospital pharmacy must draw out the hydrogen peroxide solution and mix it with the sodium hyaluronate, or a physician must do so at the patient's bedside. In the former case, the pharmacy personnel are burdened and there is risk of delay in transporting the injection mixture from the pharmacy to the patient's bedside. In the latter case, medical personnel at the patient's bedside, who are preparing the patient for radiotherapy, are burdened. In both cases, the complications of drawing out and mixing the solutions increase risks of mistakes that might compromise medical treatment or endanger the patient.

In addition, if the hydrogen peroxide solution is pre-filled using a syringe made of conventional glass (for example, borosilicate glass), the glass syringe expands during storage of the hydrogen peroxide solution and the gasket thereof is pushed back. This might interfere with the long-term storage of the hydrogen peroxide solution in such a glass syringe. To solve these problems, the present invention provides a pre-filled syringe for hydrogen peroxide solution that can be stored for a long time.

### MEANS FOR SOLVING THE PROBLEMS

According to the present invention, there is provided a pre-filled syringe for medical usage filled with a hydrogen peroxide solution and a kit comprising the pre-filled syringe as defined in the appending independent claims. The pre-filled syringe includes a barrel wherein the inner surface thereof directly contacting the hydrogen peroxide solution is made of cycloolefin polymer (COP) or cycloolefin copolymer (COC).

By using the syringe, it is possible to limit the decomposition of hydrogen peroxide in the hydrogen peroxide solution. Thus, using the pre-filled syringe, a hydrogen peroxide solution can be stored for a long time.

The hydrogen peroxide solution may include hydrogen peroxide and water.

The hydrogen peroxide solution may include an additive.

The syringe may further include a nozzle attached to the needle mounting part of the syringe.

When the syringe is already equipped with a nozzle, rapid administration is possible.

The nozzle may include a nozzle part and an adapter part connected to the needle mounting part of the syringe.

The nozzle part may be a needle or a spray nozzle.

The needle may have a groove in an echogenic pattern on an outer surface thereof.

The syringe may further include a protector. The nozzle part may be covered with the protector.

The protector may include a support member, a nozzle protection part connected to one end of the support member, and an engagement part connected to the other end of the support member.

The nozzle protection part may include a space capable of accommodating the nozzle part.

The engagement part may include a movable part and may be movably connected to the other end of the support member via the movable part.

The space may be located along the inner surface of the lateral wall of the nozzle protection part.

The support member may include a first arm, a second arm, a first movable part, a second movable part, and a third movable part.

One end of the first arm may be movably connected to the nozzle protection part via the first movable part. The other end of the first arm may be movably connected to the one end of the second arm via the second movable part. The one end of the second arm may be movably connected to the engagement part via the third movable part.

The movable part may include a rail part connected to the nozzle protection part and a rail holding part connected to the engagement part.

The rail holding part may hold the rail part slidably.

The space may be positioned inside the nozzle protection part.

The nozzle protection part may have a hollow structure.

The pre-filled syringe may further include a syringe pump.

Concentration of the hydrogen peroxide in the hydrogen peroxide solution may be 0.01 to 40% (w/v).

The present invention further provides a kit including the pre-filled syringe and the nozzle.

By using the kit, it is not necessary to choose the nozzle for the syringe. As a result, rapid administration can be facilitated.

The kit may further include a protector for covering the nozzle part.

### EFFECT OF THE INVENTION

According to the present invention, it is possible to provide a pre-filled syringe capable of storing a hydrogen peroxide solution for a long period of time until it can be used as a radiosensitizer. As a result, rapid administration can be performed. Moreover, safe administration can be performed by providing a nozzle with a protector. By using a needle as the nozzle, the hydrogen peroxide solution can be administered easily and safely. Alternatively, by using a spray nozzle as the nozzle, the hydrogen peroxide solution can be administered by spraying easily and safely. By using a syringe pump, the pre-filled syringe can be operated stably, delivering the pre-filled solution at a pre-determined appropriate rate.

### BRIEF DESCRIPTION OF THE DRAWINGS

FIG. 1 shows a schematic view of a pre-filled syringe containing a hydrogen peroxide solution according to the present embodiment.
FIG. 2 shows a pre-filled syringe with a needle according to the present embodiment.
FIG. 3 shows a pre-filled syringe with a spray nozzle according to the present embodiment.
FIG. 4 is a partially enlarged cross-sectional view of the spray nozzle according to the present embodiment.
FIG. 5 shows a pre-filled syringe with a protector according to the present embodiment.
FIG. 6 shows a pre-filled syringe with a movable protector in a protection mode according to the present embodiment.
FIG. 7 shows a pre-filled syringe with a movable protector in an administration mode according to the present embodiment.
FIG. 8 shows a pre-filled syringe with a lifting protector in a protection mode according to the present embodiment.
FIG. 9 shows a pre-filled syringe with a slide-type protector in an administration mode according to the present embodiment.
FIG. 10 shows a pre-filled syringe equipped with a syringe pump according to the present embodiment.
FIG. 11 is a schematic diagram illustrating the operation of a syringe pump according to the present embodiment.
FIG. 12 shows a graph of residual rates of hydrogen peroxide of each syringe material in the example.

### DESCRIPTION OF THE EMBODIMENTS

### Definition

For convenience, certain terms employed in the context of the present disclosure are collected here. Unless defined otherwise, all technical and scientific terms used herein have the same meaning as commonly understood by one of the ordinary skilled in the art to which this invention belongs. The singular forms "a", "and", and "the" are used herein to include plural referents unless the context clearly dictates otherwise.

Notwithstanding that the numerical ranges and parameters setting forth the broad scope of the invention are approximations, the numerical values set forth in the specific examples are described as precisely as possible. Any numerical value, however, inherently contains certain errors necessarily resulting from the standard deviation found in the respective testing measurements. Also, as used herein, the term "about" generally means within 10, 5, 1, or 0.5% of a given value or range. Alternatively, the term "about" means within an acceptable standard error of the mean when considered by one of ordinary skill in the art.

"Protection mode" in the present specification herein means a state in which skin cannot access a tip of a nozzle part by a protector, that is, a state where administration cannot be performed. "Administration mode" used in the present specification means a state in which the tip of the nozzle part is exposed from the protector, that is, a state where administration can be performed.

Hereinafter, embodiments of the present invention are illustrated in detail. The following embodiments are illustrative only and do not limit the scope of the present invention. In order to avoid redundancy, explanation for similar contents is not repeated.

### Syringe

A syringe according to the present embodiment includes a portion thereof directly contacting a hydrogen peroxide solution, in which the portion is made of cycloolefin polymer (COP) or cycloolefin copolymer (COC).

The syringe may be suitable for prefilling with a hydrogen peroxide solution. The syringe may further include the hydrogen peroxide solution in the syringe. The hydrogen peroxide solution may include hydrogen peroxide and water. The hydrogen peroxide solution may include an additive.

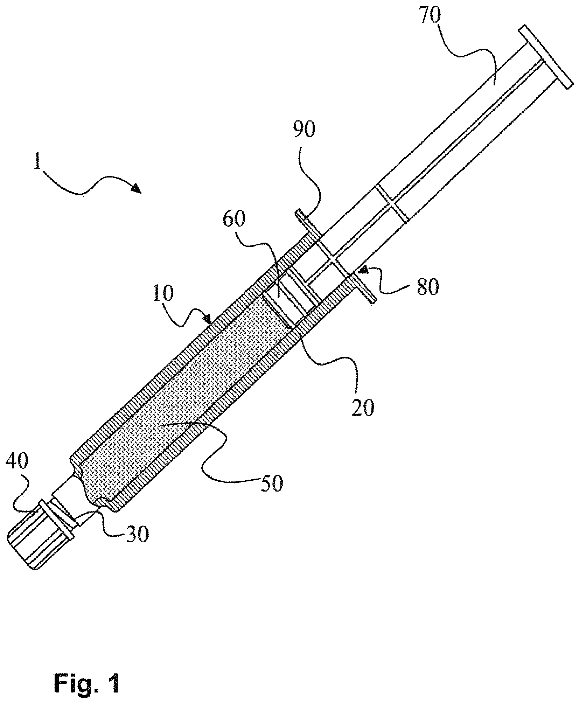

FIG. 1 shows a schematic diagram of a pre-filled syringe (1) filled with hydrogen peroxide solution (50) according to the present invention. A syringe (10), particularly a barrel (20) of the syringe (10), has generally cylindrical shape. The syringe (10) has, at one end thereof, a needle mounting part (30) from which the hydrogen peroxide solution (50) is discharged. The syringe (10) has, at the other end thereof, a rod-inserted part (80) for inserting a plunger rod (70). The syringe (10) has a flange (90) provided around the rod-inserted part (80). In order to seal the filled hydrogen peroxide solution (50), the pre-filled syringe (1) shown in FIG. 1 has a cap (40) provided on the needle mounting part (30) and the plunger rod (70) inserted from the rod-inserted part (80), the plunger rod (70) having a gasket (60).

In the present embodiment, the syringe for hydrogen peroxide solution means a syringe having a low decomposition capability of hydrogen peroxide in the hydrogen peroxide solution. In the present invention, the hydrogen peroxide solution means a solution in which a solvent (for example, water) contains hydrogen peroxide and if necessary, additives (for example, phosphoric acid and phenacetin, other than gel substrate). In an embodiment, the hydrogen peroxide solution is substantially free of the gel substrate (e.g., hyaluronic acid, salt of hyaluronic acid, hydrogel and gelatin). "Substantially free" means, for example, that the concentration of the gel substrate in the solution is less than 0.1% by mass, less than 0.05% by mass, less than 0.01% by mass, less than 0.005% by mass or less than 0.001% by mass or less than 0.1%(w/v), less than 0.05%(w/v), less than 0.01%(w/v), less than 0.005%(w/v) or less than 0.001 % (w/v).

In another embodiment, the hydrogen peroxide solution does not include the gel substrate. In the present embodiment, the syringe may be manufactured from a single material or may be made with a plurality of materials (including a multilayer structure such as a coating). In the case of the syringe manufactured from the single material, the entire syringe is made of plastic such as COP and COC. In the case of the syringe made with a plurality of materials, the part where the syringe contacts with the hydrogen peroxide solution directly (i.e. the inner surface of the barrel (20)) is made of the plastic, the remaining part may be made of a material having high decomposition capability of the hydrogen peroxide such as glass. In addition, all parts that come into contact with the hydrogen peroxide solution do not need to be made of the plastic.

Thus, the main part including the inner surface of the barrel of the syringe is made of the plastic. In other words, parts that may come into contact with the hydrogen peroxide solution, such as a plunger rod, luer lock, cap and gasket, do not need to be made of the plastic. In addition, a lubricant such as silicone oil may be applied to the inner surface of the barrel of the syringe body.

The decomposition capability of hydrogen peroxide can be determined from the ratio of the concentration of hydrogen peroxide in the hydrogen peroxide solution after start of storage under a specific temperature condition and for a specific storage period to the concentration of hydrogen peroxide in the hydrogen peroxide solution before the start of the storage (residual rate of hydrogen peroxide). The storage is performed in a sealed state. The temperature condition is not limited, but may be 35°C, 37°C, 40°C, or 60°C. The period of the storage is not limited, but may be one week, two weeks, three weeks, or four weeks, or four weeks or more. The concentration of hydrogen peroxide in the hydrogen peroxide solution before the start of the storage may be any concentration, for example in the range of 0.01 to 40% (w/v). In an embodiment, the decomposition capability of hydrogen peroxide to the plastic is lower than that of a glass. The residual rate of hydrogen peroxide in the plastic may be 70% or more, preferably 75% or more, more preferably 78% or more, still more preferably 80% or more under the condition that a solution containing 2.5 to 3.5% (w/v) hydrogen peroxide is hermetically stored at 60 °C for 4 weeks. The amount of hydrogen peroxide in the hydrogen peroxide solution can be determined by titration with a potassium permanganate solution according to an oxydol determination method described in the Japanese Pharmacopoeia.

In the present embodiment, the plastic may include COP, COC and polypropylene, but is not limited to them as long as the plastic has a lower decomposition capability of hydrogen peroxide than glass.

### Nozzle

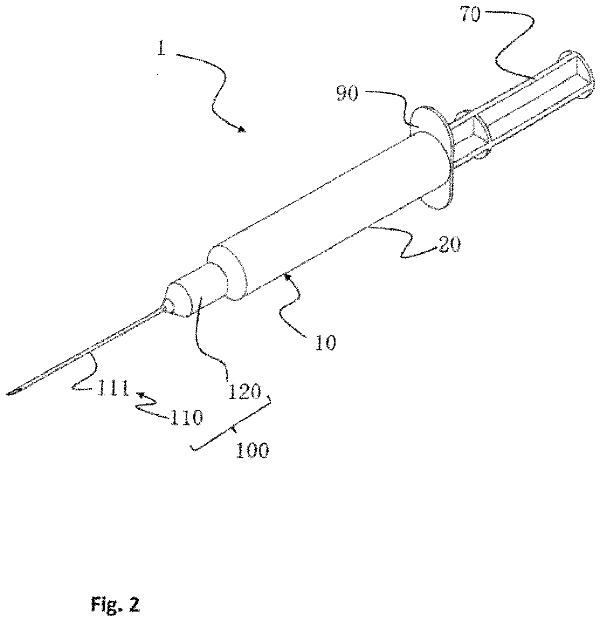

FIG. 2 shows the pre-filled syringe (1) with a nozzle (100). The nozzle (100) is attached to the needle mounting part (30) of the pre-filled syringe (1). The nozzle (100) may be pre-mounted on the needle mounting part (30) of the pre-filled syringe (1) or may be included in a kit including the pre-filled syringe (1). When the nozzle (100) is pre-mounted on the needle mounting part (30) of the pre-filled syringe (1), the nozzle (100) (or pre-filled syringe (1)) preferably includes a blocking mechanism that prevents leakage of the hydrogen peroxide solution (50) until the pre-filled syringe (1) is used.

The nozzle (100) includes a nozzle part (110) and an adapter part (120) connected to the nozzle part (110). The adapter part (120) is connected to the needle mounting part (30) of the pre-filled syringe (1). The inside of nozzle part (110) is in fluid communication with the inside of the adapter part (120).

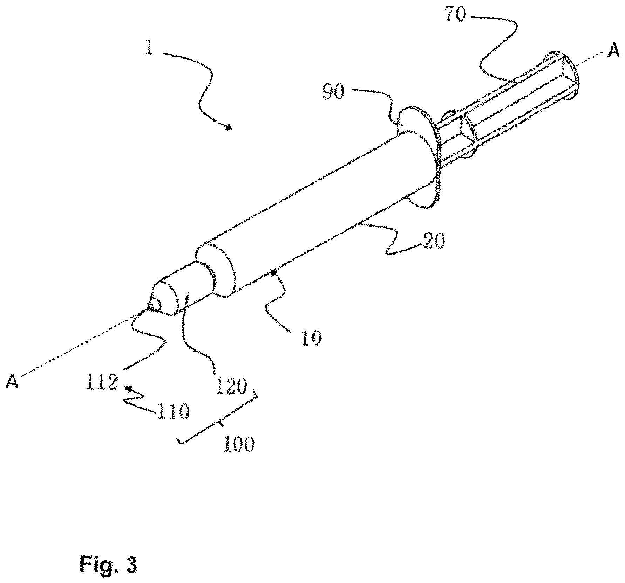

The pre-filled syringe 1 shown in FIG. 2 includes a needle 111 as the nozzle part 110. In another embodiment, the pre-filled syringe 1 includes a spray nozzle 112 as the nozzle part 110 (FIG. 3). The spray nozzle 112 shown in FIG. 3 is integrally molded in the adapter part 120. In another embodiment, the spray nozzle 112 is detachably attached to the adapter part 120.

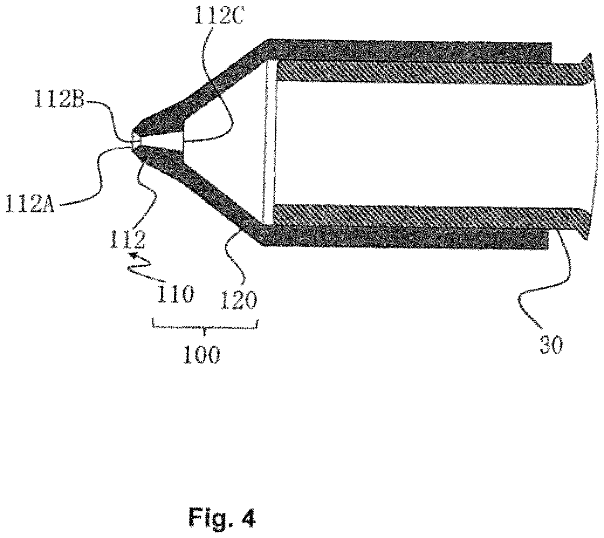

FIG. 4 shows a partial enlarged cross-sectional view of the spray nozzle (112) shown in FIG. 3. The cross-sectional view of FIG.4 represents a cross section of the pre-filled syringe (1) shown in FIG. 3 passing through the central axis A-A. The spray nozzle (112) includes an outlet (112A), an orifice (112B), and an inlet (112C). The inner diameter of the outlet (112A) according to the present embodiment decreases from the outside of the spray nozzle (112) toward the orifice (112B). The inner diameter of the inlet (112C) according to the present embodiment decreases from the inside of the spray nozzle (112) toward the orifice (112B). Depending on the desired particle size of hydrogen peroxide solution (50), the inner diameter of the orifice (112B) can be varied. The inner diameter of the outlet (112A) may be the same as the inner diameter of the orifice (112B), and the inner diameter of the inlet (112C) may be the same as the inner diameter of the orifice (112B). The inner diameter of the spray nozzle (112) may be constant.

The needle (111) may have a groove with an echogenic pattern groove on an outer surface thereof. The echogenic pattern is not particularly limited as long as it is the groove pattern that improves the visibility of the needle (111) even under an ultrasonic image.

### Protector

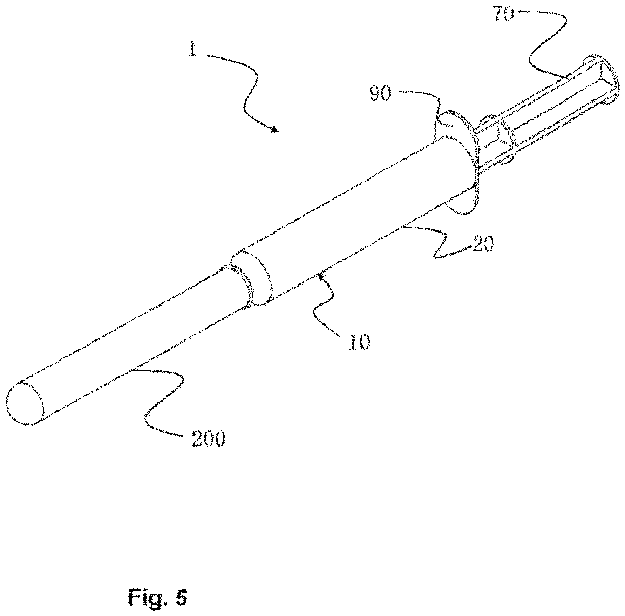

FIG. 5 shows the pre-filled syringe (1) equipped with a protector (200). The protector (200) can cover the nozzle (100). The protector (200) may be detachably connected to the adapter part (120) (or pre-filled syringe (1)) of the nozzle (100) by fitting or screwing. The protector (200) may include a blocking mechanism that prevents leakage of the hydrogen peroxide solution (50) of the pre-filled syringe (1). The blocking mechanism can prevent leakage of the hydrogen peroxide solution (50) of the pre-filled syringe (1), for example, by contacting the inside of the tip of the protector (200) with the tip of the nozzle (100).

### Movable protector

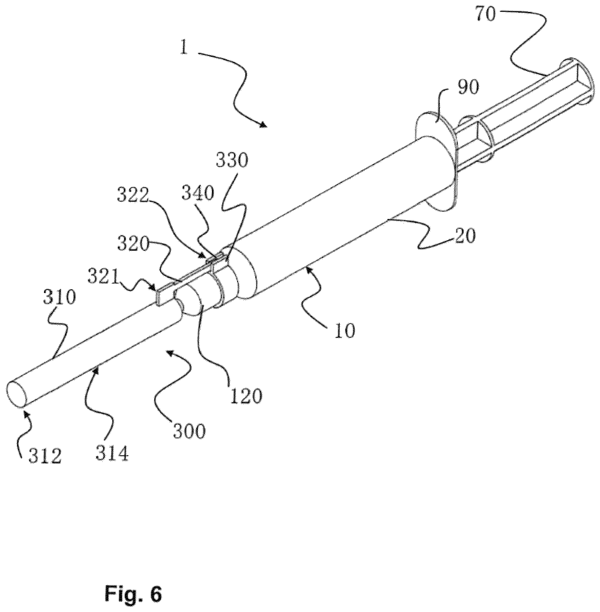

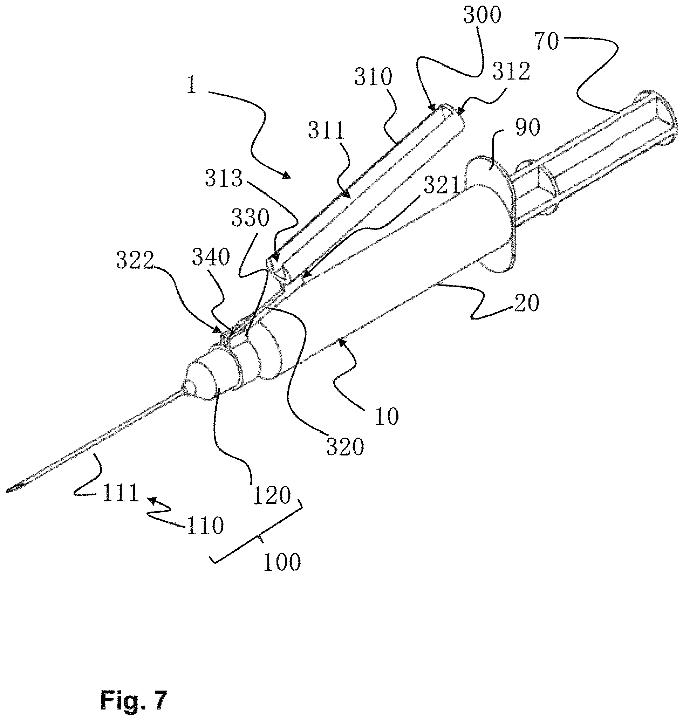

FIGs. 6 and 7 show the pre-filled syringe 1 equipped with a moveable protector (300). The movable protector (300) shown in FIG. 6 protects the nozzle part 110. The movable protector (300) shown in FIG. 7 is positioned so as to expose the nozzle part (110).

The movable protector (300) includes a support member (320), a nozzle protection part (310) connected to one end (321) of the support member (320), and an engagement part (330) connected to the other end of (322) of the support member (320). The engagement part (330) is connected to the other end (322) of the support member (320) via a movable part (340). The engagement part (330) is detachably connected to the adapter part (120).

The nozzle protection part (310) of the movable protector (300) has a space that can accommodate the nozzle part (110) of the nozzle (100) in the nozzle protection part (310). In the present embodiment, the space is groove (311). The groove (311) is formed on a side wall (314) of the nozzle protection part (310). A front end (312) of the nozzle protection part (310) is closed. A rear end (313) of the nozzle protection part (310) is open.

The movable protector (300) can expose the nozzle part (110) of the nozzle (100) from the groove (311) of the nozzle protection part (310) by rotating the movable protector (300) around the movable part (340) as a rotation axis without physically separating the movable protector (300) from the adapter part (120), and vice versa.

The movable protector (300) may include a plurality of the support members (320). When the movable protector (300) includes a plurality of the support members (320), each of the support members (320) may be connected via a movable part. The engagement part (330) may be engaged with the adapter part (120) of the nozzle (100) or the pre-filled syringe (1). In the present embodiment, the movable part (340) is a pivot part including a shaft but is not limited thereto. The movable part (340) may be a bent part that can be bent. When the movable part (340) is the bent part, the support member (320) and the engagement part (330) may be integrally formed.

### Slide-type protector

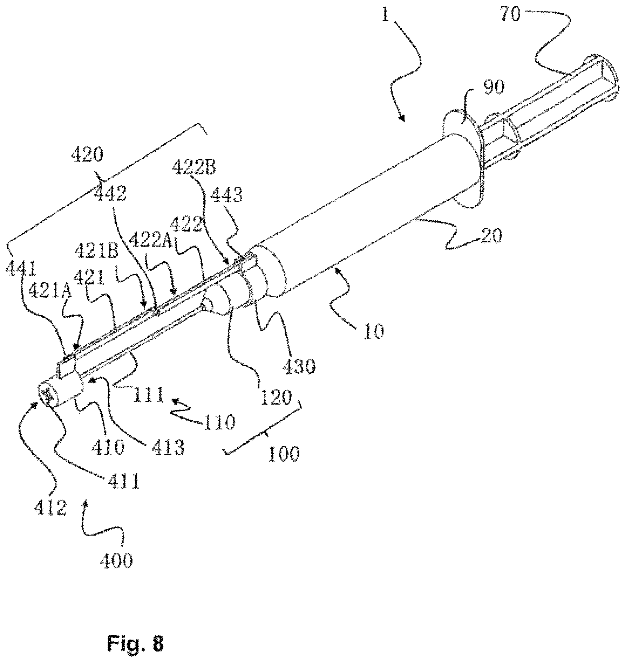

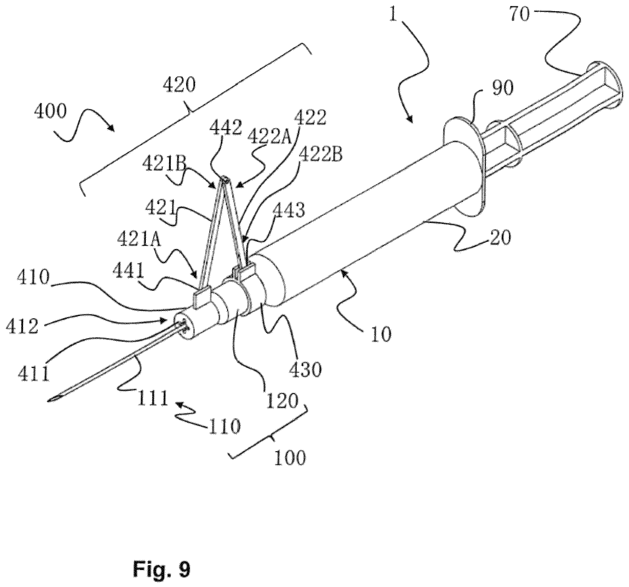

FIGs. 8 and 9 show the pre-filled syringe 1 equipped with a slide-type protector (400). The slide-type protector (400) shown in FIG. 8 protects the nozzle part (110). The slide-type protector (400) shown in FIG. 9 is positioned so as to expose the nozzle part (110).

The slide-type protector (400) includes a support member (420), a nozzle protection part (410) connected to one end of the support member (420), and an engagement part (430) connected to the other end of the support member (420). The engagement part (430) is detachably connected to the adapter part (120). The support member (420) includes a first arm (421), a second arm (422), a first movable part (441), a second movable part (442), and a third movable part (443). In the present embodiment, the nozzle protection part (410) has a hollow structure, and has a space that can accommodate a front end of the nozzle part (110) from a rear end (413) of the nozzle protection part (410). When the slide-type protector (400) is in a protection mode, the front end of the nozzle part (110) is accommodated in the nozzle protection part (410). When the slide-type protector (400) is in an administration mode, the front end of the nozzle part (110) protrudes from an opening (411) of the nozzle protection part (410). In the present embodiment, the opening (411) has a cross shape, but may have another shape.

One end (421A) of the first arm (421) is movably connected to the nozzle protection part (410) via the first movable part (441). The other end (421B) of the first arm (421) is movably connected to one end (422A) of the second arm (422) via the second movable part (442). The other end (422B) of the second arm (422) is movably connected to the engagement part (430) via the third movable part (443).

By rotating each arm with respect to the central axis of each movable part so that the second movable part (442) is away from the nozzle (100), the nozzle protection part (410) can be moved in the direction of the adapter part (120), resulting that the nozzle part (110) of the nozzle (100) can be exposed from the opening (411) of the nozzle protection part (410) without physically separating the slide-type protector (400) from the adapter part (120), and vice versa.

The number of the arms and the number of the movable parts can be changed as necessary. The length of the arm can be changed according to the length of the nozzle part (110).

### Another embodiment

In another embodiment, the slide-type protector (400) includes a rail part, a nozzle protection part (410) connected to one end of the rail part, and a rail holding part connected to the other end of the rail part. The rail holding part hold the rail part slidably. The rail holding part is detachably connected to the adapter part (120). The opening (411) is provided at the front end of the nozzle protection part (410). By sliding the nozzle protection part (410) in the longitudinal direction of the pre-filled syringe (1), the nozzle part (110) can be accommodated in or exposed from the opening (411).

### Syringe pump

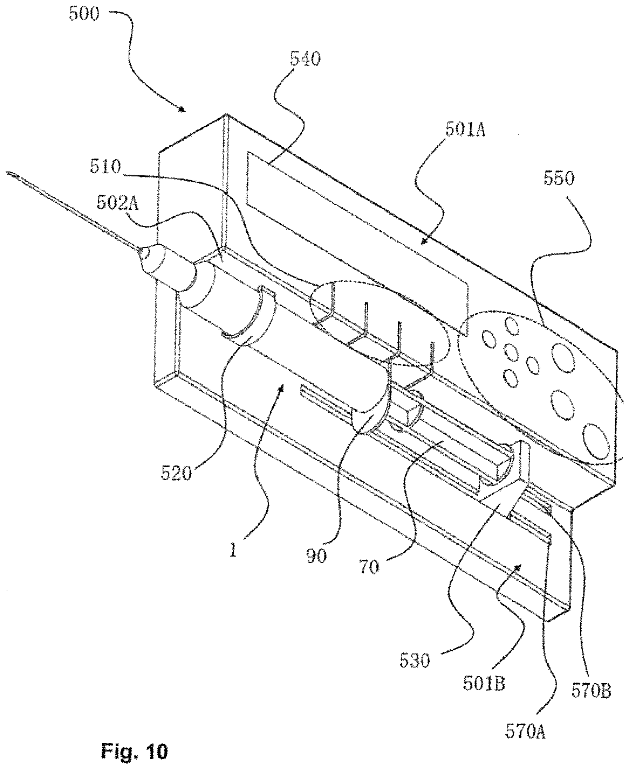

FIG. 10 shows the pre-filled syringe (1) equipped with a syringe pump (500). The syringe pump (500) in the present embodiment includes slits (510) into which one end of the flange (90) of the pre-filled syringe (1) is inserted, a holder (520) that fixes the pre-filled syringe (1), a movable wall (530) that pushes the plunger rod (70) of the pre-filled syringe (1), a monitor (540), switches (550), a processor (560), a memory (561), a pressure sensor (562), a battery (563), and an electric motor (564).

The monitor (540) and switches (550) are provided on a first surface (501A) of the syringe pump (500). The movable wall (530) is provided on a second surface (501B) of the syringe pump (500). The second surface (501B) of the syringe pump (500) is provided at a lower position than the first surface (501A) of the syringe pump (500). The first surface (501A) of the syringe pump (500) is connected to the second surface (501B) of the syringe pump (500) via the first wall (502A) of the syringe pump (500). The slits (510) are formed so as to pass through the first surface (501A) and first wall (502A) of the syringe pump (500).

The movable wall (530) is connected to two threaded rods (570A) and (570B) provided on the second surface (501B). When the threaded rods (570A) and (570B) are rotated by, for example, the electric motor (564), the movable wall (530) can move in the direction of pushing (or pulling) the plunger rod (70) of the pre-filled syringe (1). The movable wall (530) of the syringe pump (500) is electrically driven but may be mechanically driven.

When the syringe pump (500) is used, the one end of the flange (90) of the pre-filled syringe (1) is inserted into any one of the slits (510). By inserting the one end of the flange (90) of the pre-filled syringe (1) into the slit (510), the movement of the pre-filled syringe (1) in the moving direction of the movable wall (530) is prevented. Depending on the length of the pre-filled syringe (1), any one of the slits (510) can be selected.

The pre-filled syringe (1) is further fixed by the holder (520). The holder (520) is configured to press the pre-filled syringe (1) against the first wall (502A) and second surface (501B). By fixing the pre-filled syringe (1) using the holder (520), the pre-filled syringe (1) is prevented from falling off the syringe pump (500).

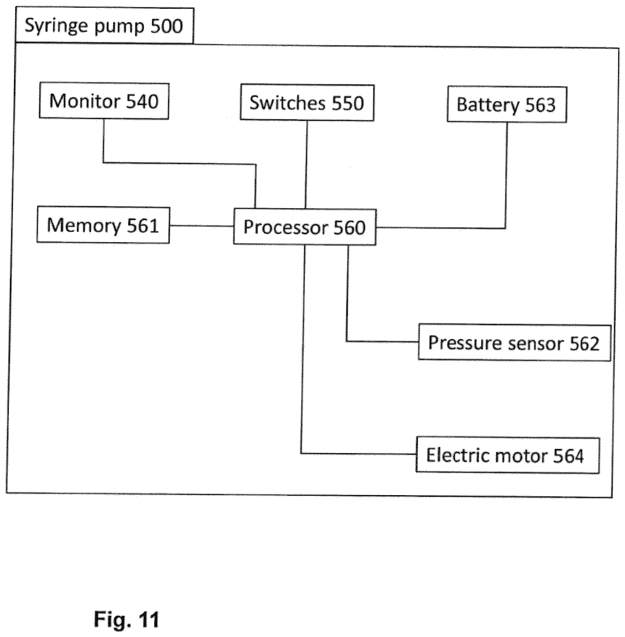

Operation of the syringe pump (500) will be described with reference to FIG. 11. The syringe pump (500) is moved by the battery (563) power. The operation of the syringe pump (500) can be set by operating the switches (550). Requests from the switches (550) are processed by the processor (560). The processor (560) can read out necessary information (such as a program) from the memory (561) upon the request and can store the necessary information in the memory (561). The processor (560) can display a processing result on the monitor (540). When the processor (560) receives a request to move the syringe pump (500), the processor (560) processes the request so that the electric motor (564) rotates. Based on the information of the pressure sensor (562) connected to the movable wall (530), the processor (560) can process the information so that the electric motor (564) stops.

The syringe pump (500) can set a flow rate, administration time, an inner diameter of the syringe, a pressure threshold, and the like, thereby enabling stable administration.

### kit

In yet another embodiment, a kit including: the syringe and the nozzle is provided.

The kit includes the pre-filled syringe (1) and nozzle (100). The kit may include a plurality of the pre-filled syringes (1) and nozzles (100). The kit may include the protector (200), (300) or (400) that covers the nozzle (100). The kit may include additional elements (e.g., instructions or dosing schedules) for treatment of tumors with anti-cancer drug or radiation.

In the present embodiment, the concentration of hydrogen peroxide in the hydrogen peroxide solution in the pre-filled syringe is, for example, 0.01, 0.02, 0.03, 0.04, 0.05, 0.06, 0.07, 0.08, 0.09, 0.1, 0.5, 1, 5, 10, 15, 20, 25, 30, 35 or 40%, or may be in the range between any two of the numerical values exemplified herein, for example, 0.01 to 40% (w/v), preferably, 0.05 to 30% (w/v).

### Materials

The material of the nozzle part (110) can be changed according to the purpose and situation of the use of the present embodiment. When the nozzle part (110) is the needle (111), the material of the nozzle part (110) (that is, the needle (111)) may be a metal such as stainless steel. The material of the adapter part (120) may be plastic (e.g., COP, COC, polypropylene, and polycarbonate), metal, rubber or glass. When the nozzle part (110) is the spray nozzle (112), the material of the nozzle part (110) (i.e., spray nozzle (112)) may be plastic (e.g., COP, COC, polypropylene and polycarbonate), metal, rubber or glass. When the spray nozzle (112) is formed integrally with the adapter part (120), the same material as that of the adapter part (120) is used.

The material of the protector (200) can include, but is not limited to, plastic (for example, COP, COC, polypropylene, and polycarbonate), metal, rubber and glass. The material of the movable protector (300) and the slide-type protector (400) may be the same as the material of the protector (200). The material of the movable parts of the movable protector (300) and the slide-type protector (400) (the movable part (340), first movable part (441), second movable part (442) and third movable part (443)) may be different from the material of other parts of the movable protector (300) and slide-type protector (400) depending on the purpose and situation of the use of the present embodiment.

A housing of the syringe pump (500) may be made of metal or plastic (for example, polycarbonate). The material of the movable wall (530) may be the same as or different from the material of the housing of the syringe pump (500). The holder (520) may be made of metal, rubber or plastic. The threaded rods (570A) and (570B) are preferably made of metal but may be plastic.

### EXAMPLES

### Hydrogen peroxide solution stability test

A stability test of a hydrogen peroxide solution was performed using a glass syringe, a COP syringe, and a COC syringe. 1 mL of the hydrogen peroxide solution was added to each syringe, sealed, and then stored at 60 °C for 4 weeks. The residual rates of hydrogen peroxide in the hydrogen peroxide solutions after storage were measured. Oxydol "KENEI" (containing 2.5 to 3.5%(w/v) hydrogen peroxide, phosphoric acid and phenacetin) manufactured by Kenei Pharmaceutical Co., Ltd. was used as the hydrogen peroxide solution. The amount of hydrogen peroxide in the hydrogen peroxide solution was detected by titration with a potassium permanganate solution according to an oxydol determination method described in the Japanese Pharmacopoeia.

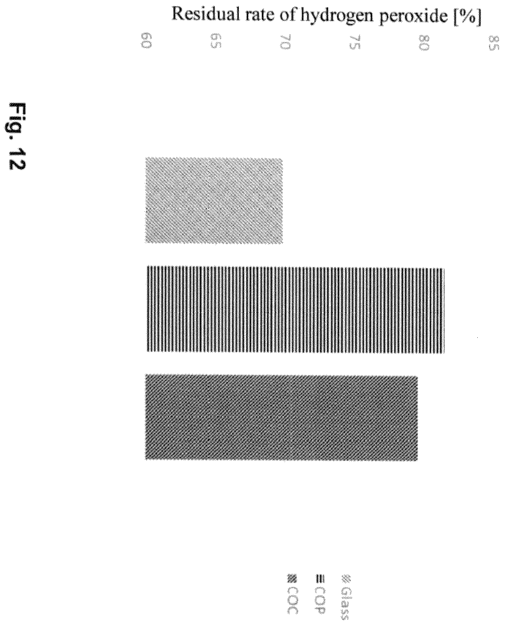

The results are shown in FIG. 12. In the case of the glass, the residual rate of hydrogen peroxide was less than 70%, while the residual rate regarding COP and COC were 70% or more. As a result, the COP and COC syringes were able to suppress the decomposition of hydrogen peroxide more than the glass syringe.

### EXPLANATION OF REFERENCES

- 1: Pre-filled syringe
- 10: Syringes
- 20: Barrel
- 30: Needle mounting part
- 40: Cap
- 50: Hydrogen peroxide solution
- 60: Gasket
- 70: Plunger rod
- 80: Rod-inserted part
- 90: Flange
- 100: Nozzle
- 110: Nozzle part
- 111: Needle
- 112: Spray nozzle
- 112A: Outlet
- 112B: Orifice
- 112C: Inlet
- 120: Adapter part
- 200: Protector
- 300: Movable protector
- 310: Nozzle protection part
- 311: Groove
- 312: Front end of nozzle protection part
- 313: Rear end of nozzle protection part
- 314: Side wall of nozzle protection part
- 320: Support member
- 321: One end of support member
- 322: Other end of support member
- 330: Engagement part
- 340: Movable part
- 400: Slide-type protector
- 410: Nozzle protection part
- 411: Opening
- 412: Front end of nozzle protection part
- 413: Rear end of nozzle protection part
- 420: Support member
- 421: First arm
- 421A: One end of first arm
- 421B: Other end of first arm
- 422: Second arm
- 422A: One end of second arm
- 422B: Other end of second arm
- 430: Engagement part
- 441: First movable part
- 442: Second movable part
- 443: Third movable part
- 500: Syringe pump
- 501A: First surface of syringe pump
- 501B: Second surface of syringe pump
- 502A: First wall of syringe pump
- 510: Slit
- 520: Holder
- 530: Movable wall
- 540: Monitor
- 550: Switch
- 560: Processor
- 561: Memory
- 562: Pressure sensor
- 563: Battery
- 564: Electric motor
- 570A, 570B: Threaded rod

## Claims

1. A pre-filled syringe (1) for medical usage filled with a hydrogen peroxide solution (50), comprising:
a syringe (10) with:
a plunger rod (70) having a gasket (60),
a barrel (20) having a cylindrical shape,
a needle mounting part (30) provided at one end of the syringe (10) for discharging the hydrogen peroxide solution (50) therefrom,
a cap (40) provided on the needle mounting part (30) for sealing the filled hydrogen peroxide solution (50),
a rod-inserted part (80) provided at the other end of the syringe (10) for inserting the plunger rod (70), and
a flange (90) provided around the rod-inserted part (80),
**characterized in that**
the inner surface of the barrel (20) is made of cycloolefin polymer (COP) or cycloolefin copolymer (COC), and
the syringe (10) is filled with the hydrogen peroxide solution (50) therein.

2. The pre-filled syringe (1) according to claim 1,
wherein the hydrogen peroxide solution (50) comprises hydrogen peroxide and water.

3. The pre-filled syringe (1) according to claim 2, wherein the hydrogen peroxide solution (50) comprises an additive.

4. The pre-filled syringe (1) according to any one of claims 1 to 3, further comprising a nozzle (100) attached to the needle mounting part (30) of the syringe (10).

5. The pre-filled syringe (1) according to claim 4, wherein the nozzle (100) comprises a nozzle part (110) and an adapter part (120) connected to the needle mounting part (30) of the syringe (10), and the nozzle part (110) is a needle (111) or a spray nozzle (112).

6. The pre-filled syringe (1) according to claim 5, wherein the needle (111) has a groove with an echogenic pattern groove on an outer surface thereof.

7. The pre-filled syringe (1) according to claim 5 or 6, further comprising a protector (200), wherein the nozzle part (110) is covered with the protector (200, 300, 400).

8. The pre-filled syringe (1) according to claim 7, wherein the protector (300) comprises a support member (320), a nozzle protection part (310) connected to one end (321) of the support member (320), and an engagement part (330) connected to the other end (322) of the support member (320), and
the nozzle protection part (310) comprises a space capable of accommodating the nozzle part (110).

9. The pre-filled syringe (1) according to claim 8, wherein the engagement part (330) comprises a movable part (340) and is movably connected to the other end (322) of the support member (320) via the movable part (340).

10. The pre-filled syringe (1) according to claim 8 or 9, wherein the space is positioned on a side wall (314) of the nozzle protection part (310).

11. The pre-filled syringe (1) according to claim 8, wherein the support member (320) comprises a first arm (421), a second arm (422), a first movable part (441), a second movable part (442), and a third movable part (443),
one end (421A) of the first arm (421) is movably connected to the nozzle protection part (310) via the first movable part (441),
the other end (421B) of the first arm (421) is movably connected to one end (422A) of the second arm (422) via the second movable part (442), and
the other end (422B) of the second arm (422) is movably connected to the engagement part (430) via the third movable part (443).

12. The pre-filled syringe (1) according to claim 9, wherein the movable part (340) comprises a rail part connected to the nozzle protection part (410) and a rail holding part connected to the engagement part (430), and
the rail holding part holds the rail part slidably.

13. The pre-filled syringe (1) according to claim 11 or 12, wherein the space is positioned inside the nozzle protection part (410), and
the nozzle protection part (410) has a hollow structure.

14. The pre-filled syringe (1) according to any one of claims 1 to 13, further comprising a syringe pump (500).

15. The pre-filled syringe (1) according to any one of claims 1 to 14, wherein the concentration of the hydrogen peroxide in the hydrogen peroxide solution (50) is 0.01 to 40% (w/v).

16. A kit comprising: the pre-filled syringe (1) according to claim 1, and a nozzle (100).

17. The kit according to claim 16, further comprising a protector (200, 300, 400) for covering the nozzle part (100).

## Patentansprüche

1. Fertigspritze (1) zur medizinischen Verwendung, die mit einer Wasserstoffperoxid-Lösung (50) gefüllt ist, umfassend:
eine Spritze (10) mit:
einem Stempel (70) mit einer Dichtung (60),
einem Zylinder (20) mit einer zylindrischen Form,
einem Nadelbefestigungsteil (30), der an einem Ende der Spritze (10) zum Auslassen der Wasserstoffperoxid-Lösung (50) daraus vorgesehen ist,
einer Kappe (40), die auf dem Nadelbefestigungsteil (30) zum Abdichten der eingefüllten Wasserstoffperoxid-Lösung (50) vorgesehen ist,
einem Stempeleinsatzteil (80), der an dem anderen Ende der Spritze (10) zum Einsetzen des Stempels (70) vorgesehen ist, und
einem Flansch (90), der um den Stempeleinsatzteil (80) herum vorgesehen ist,
**dadurch gekennzeichnet, dass**
die Innenfläche des Zylinders (20) aus Cycloolefin-Polymer (COP) oder Cycloolefin-Copolymer (COC) hergestellt ist, und
die Spritze (10) mit der Wasserstoffperoxid-Lösung (50) darin befüllt ist.

2. Fertigspritze (1) nach Anspruch 1,
wobei die Wasserstoffperoxid-Lösung (50) Wasserstoffperoxid und Wasser umfasst.

3. Fertigspritze (1) nach Anspruch 2, wobei die Wasserstoffperoxid-Lösung (50) ein Additiv umfasst.

4. Fertigspritze (1) nach einem der Ansprüche 1 bis 3, ferner umfassend eine Düse (100), die an dem Nadelbefestigungsteil (30) der Spritze (10) angebracht ist.

5. Fertigspritze (1) nach Anspruch 4, wobei die Düse (100) einen Düsenteil (110) und einen Adapterteil (120) umfasst, der mit dem Nadelbefestigungsteil (30) der Spritze (10) verbunden ist, und der Düsenteil (110) eine Nadel (111) oder eine Sprühdüse (112) ist.

6. Fertigspritze (1) nach Anspruch 5, wobei die Nadel (111) eine Nut mit einem echogenen Muster an einer Außenfläche davon aufweist.

7. Fertigspritze (1) nach Anspruch 5 oder 6, ferner umfassend ein Schutzelement (200), wobei das Düsenteil (110) mit dem Schutzelement (200, 300, 400) bedeckt ist.

8. Fertigspritze (1) nach Anspruch 7, wobei das Schutzelement (300) ein Stützelement (320), einen Düsenschutzteil (310), der mit einem Ende (321) des Stützelements (320) verbunden ist, und einen Ineingriffnahmeteil (330), der mit dem anderen Ende (322) des Stützelements (320) verbunden ist, umfasst, und
der Düsenschutzteil (310) einen Raum umfasst, der in der Lage ist, den Düsenteil (110) aufzunehmen.

9. Fertigspritze (1) nach Anspruch 8, wobei der Ineingriffnahmeteil (330) einen beweglichen Teil (340) umfasst und über den beweglichen Teil (340) beweglich mit dem anderen Ende (322) des Stützelements (320) verbunden ist.

10. Fertigspritze (1) nach Anspruch 8 oder 9, wobei der Raum an einer Seitenwand (314) des Düsenschutzteils (310) angeordnet ist.

11. Fertigspritze (1) nach Anspruch 8, wobei das Stützelement (320) einen ersten Arm (421), einen zweiten Arm (422), einen ersten beweglichen Teil (441) und einen zweiten beweglichen Teil (442) und einen dritten beweglichen Teil (443) umfasst,
ein Ende (421A) des ersten Arms (421) über den ersten beweglichen Teil (441) beweglich mit dem Düsenschutzteil (310) verbunden ist,
das andere Ende (421B) des ersten Arms (421) über den zweiten beweglichen Teil (442) beweglich mit einem Ende (422A) des zweiten Arms (422) verbunden ist und
das andere Ende (422B) des zweiten Arms (422) über den dritten beweglichen Teil (443) beweglich mit dem Ineingriffnahmeteil (430) verbunden ist.

12. Fertigspritze (1) nach Anspruch 9, wobei der bewegliche Teil (340) einen Schienenteil, der mit dem Düsenschutzteil (410) verbunden ist, und einen Schienenhalteteil, der mit dem Ineingriffnahmeteil (430) verbunden ist, umfasst, und
der Schienenhalteteil den Schienenteil gleitend hält.

13. Fertigspritze (1) nach Anspruch 11 oder 12, wobei der Raum innerhalb des Düsenschutzteils (410) angeordnet ist und
der Düsenschutzteil (410) eine hohle Struktur aufweist.

14. Fertigspritze (1) nach einem der Ansprüche 1 bis 13, ferner umfassend eine Spritzenpumpe (500).

15. Fertigspritze (1) nach einem der Ansprüche 1 bis 14, wobei die Konzentration des Wasserstoffperoxids in der Wasserstoffperoxid-Lösung (50) 0,01 bis 40 Gew.-/Vol.-% beträgt.

16. Kit, umfassend: die Fertigspritze (1) nach Anspruch 1 und eine Düse (100).

17. Kit nach Anspruch 16, ferner umfassend ein Schutzelement (200, 300, 400) zum Bedecken des Düsenteils (100).

## Revendications

1. Seringue préremplie (1) pour usage médical remplie avec une solution de peroxyde d'hydrogène (50), comprenant :
une seringue (10) avec :
une tige de piston (70) ayant un joint d'étanchéité (60),
un tube (20) ayant une forme cylindrique,
une partie de montage d'aiguille (30) fournie à une extrémité de la seringue (10) pour évacuer la solution de peroxyde d'hydrogène (50) de celle-ci,
un bouchon (40) fourni sur la partie de montage d'aiguille (30) pour sceller la solution de peroxyde d'hydrogène (50),
une partie d'insertion de tige (80) fournie sur l'autre extrémité de la seringue (10) pour insérer la tige de piston (70), et
une bride (90) fournie autour de la partie d'insertion de tige (80),
**caractérisée en ce que**
la surface intérieure du tube (20) est constituée de polymère de cyclooléfine (COP) ou de copolymère de cyclooléfine (COC), et
la seringue (10) est remplie avec la solution de peroxyde d'hydrogène (50).

2. Seringue préremplie (1) selon la revendication 1,
la solution de peroxyde d'hydrogène (50) comprenant du peroxyde d'hydrogène et de l'eau.

3. Seringue préremplie (1) selon la revendication 2, la solution de peroxyde d'hydrogène (50) comprenant un additif.

4. Seringue préremplie (1) selon l'une quelconque des revendications 1 à 3, comprenant en outre une buse (100) attachée à la partie de montage d'aiguille (30) de la seringue (10).

5. Seringue préremplie (1) selon la revendication 4, la buse (100) comprenant une partie de buse (110) et une partie d'adaptateur (120) connecté à la partie de montage d'aiguille (30) de la seringue (10), et la partie de buse (110) étant une aiguille (111) ou une buse pulvérisatrice (112).

6. Seringue préremplie (1) selon la revendication 5, l'aiguille (111) ayant une rainure avec une rainure de motif échogène sur une surface extérieure de celle-ci.

7. Seringue préremplie (1) selon l'une des revendications 5 à 6, comprenant en outre un élément protecteur (200), la partie de buse (110) étant couverte par l'élément protecteur (200, 300, 400).

8. Seringue préremplie (1) selon la revendication 7, l'élément protecteur (300) comprenant un élément support (320), une partie de protection de buse (310) connectée à une extrémité (321) de l'élément support (320), et une partie d'engagement (330) connectée à l'autre extrémité (322) de l'élément support (320), et
la partie de protection de buse (310) comprenant un espace capable d'accueillir la partie de buse (110).

9. Seringue préremplie (1) selon la revendication 8, la partie d'engagement (330) comprenant une partie déplaçable (340) et étant connectée de façon déplaçable à l'autre extrémité (322) de l'élément support (320) via la partie déplaçable (340).

10. Seringue préremplie (1) selon l'une des revendications 8 à 9, l'espace étant positionné sur une paroi latérale (314) de la partie de protection de buse (310).

11. Seringue préremplie (1) selon la revendication 8, l'élément support (320) comprenant un premier bras (421), un deuxième bras (422), une première partie déplaçable (441), une deuxième partie déplaçable (442) et une troisième partie déplaçable (443),
une extrémité (421A) du premier bras (421) étant connectée de façon déplaçable à la partie de protection de buse (310) via la première partie déplaçable (441),
l'autre extrémité (421B) du premier bras (421) étant connectée de façon déplaçable à l'une extrémité (422A) du deuxième bras (422) via la deuxième partie déplaçable (442), et
l'autre extrémité (422B) du deuxième bras (422) étant connectée de façon déplaçable à la partie d'engagement (430) via la troisième partie déplaçable (443).

12. Seringue préremplie (1) selon la revendication 9, la partie déplaçable (340) comprenant une partie de rail connectée à la partie de protection de buse (410) et une partie de maintien de rail connectée à la partie d'engagement (430), et
la partie de maintien de rail maintenant la partie de rail de façon coulissante.

13. Seringue préremplie (1) selon l'une des revendications 11 ou 12, l'espace étant positionné
à l'intérieur de la partie de protection de buse (410), et
la partie de protection de buse (410) ayant une structure creuse.

14. Seringue préremplie (1) selon l'une quelconque des revendications 1 à 13, comprenant en outre une pompe de seringue (500).

15. Seringue préremplie (1) selon l'une quelconque des revendications 1 à 14, la concentration du peroxyde d'hydrogène dans la solution de peroxyde d'hydrogène (50) étant de 0,01 % à 40 % (m/v).

16. Nécessaire, comprenant : la seringue préremplie (1) selon la revendication 1 et une buse (100).

17. Nécessaire selon la revendication 16, comprenant en outre un élément protecteur (200, 300, 400) pour couvrir la partie de buse (100).
